(19) **Europäisches Patentamt**

**European Patent Office**

**Office européen des brevets**

(11) Veröffentlichungsnummer : **0 163 749**
**B1**

(12) **EUROPÄISCHE PATENTSCHRIFT**

(45) Veröffentlichungstag der Patentschrift :
02.03.88

(51) Int. Cl.⁴ : **C 02 F   3/28, B 04 C   9/00**

(21) Anmeldenummer : 84106319.1

(22) Anmeldetag : 02.06.84

(54) Verfahren und Vorrichtung zum Trennen von Biomasse und anorganischen Bestandteilen aus dem Schlamm eines Methan-Reaktors einer anaeroben Abwasseranlage.

(43) Veröffentlichungstag der Anmeldung :
11.12.85 Patentblatt 85/50

(45) Bekanntmachung des Hinweises auf die Patenterteilung : 02.03.88 Patentblatt 88/09

(84) Benannte Vertragsstaaten :
AT CH DE FR GB IT LI NL

(56) Entgegenhaltungen :
CH-A-   109 312
CH-A-   475 919
DE-A- 3 150 073
NL-C-   58 270

(73) Patentinhaber : GEBRÜDER SULZER AKTIENGESELL-SCHAFT
Zürcherstrasse 9
CH-8401 Winterthur (CH)

(72) Erfinder : Redwanz, Jürgen
Niersstrasse 111
D-404 Neuss 21 (DE)

(74) Vertreter : Dipl.-Ing. H. Marsch Dipl.-Ing. K. Sparing
Dipl.-Phys.Dr. W.H. Röhl Patentanwälte
Rethelstrasse 123
D-4000 Düsseldorf (DE)

## Beschreibung

Die Erfindung betrifft ein Verfahren zum Trennen von Biomasse und anorganischen Bestandteilen aus dem Schlamm eines Methan-Reaktors einer anaeroben Abwasseranlage, wobei das zu trennende Schlamm/Wasser-Gemisch in Bodennähe aus dem Reaktor abgezogen und der Zulauföffnung eines Hydrozyklons zugeführt wird, wobei ferner der Oberlauf des Hydrozyklons in einen mindestens Biomasse und Wasser enthaltenden Vorlagebehälter geleitet wird, von dem aus schliesslich Schlamm/Wasser-Gemisch in den Reaktor zurückgefördert wird. Weiterhin betrifft die Erfindung eine Vorrichtung zur Durchführung des Verfahrens.

Bekanntlich ist es notwendig, anorganische Bestandteile, vor allem ausgefallenes Kalzium-Carbonat, aus dem Schlamm von Methan-Reaktoren anaerober Abwasseranlagen zu entfernen, sofern diese Bestandteile bestimmte, in den einzelnen Anlagen unter Umständen unterschiedliche Grenzwerte überschreiten.

Eine Möglichkeit für die Beseitigung der anorganischen Bestandteile besteht darin, einen Teil des im Methan-Reaktor anfallenden Schlammes als Ganzes aus der Anlage wegzuführen. Dabei geht jedoch eine unzulässig grosse Menge an anaerober Biomasse verloren.

Es sind daher Verfahren und Vorrichtungen bekannt, um mindestens einen Teil der anorganischen Bestandteile vom Wasser und der Biomasse zu trennen und so aus dem Anaerobie-Prozess auszuscheiden. Die dabei anfallende Biomasse wird zusammen mit dem Wasser in den Methan-Reaktor zurückgeführt. So ist beispielsweise aus der CH-A-475 919 ein Verfahren bekannt, bei dem der Inhalt eines Faulbehälters in einem Zyklon entsandet wird, wobei ein Teil des entsandeten Schlamms in den Behälter zurückgeführt, der andere Teil jedoch in einer Zentrifuge entwässert und aus dem Prozess ausgeschieden wird, so dass ein Teil der Biomasse verlorengeht. Die zugehörige Anlage enthält einen Entsandungskreislauf, in dem ein Zyklon vorgesehen ist; dessen Oberlauf ist einem Standrohr verbunden, von dem aus eine erste Leitung zurück in den Behälter und eine zweite zu der Zentrifuge führen. Die Zuführung des zu entsandenden Schlammes zu dem Zyklon erfolgt dabei mit Hilfe einer Pumpe; die gegen « mechanische » Belastungen empfindliche Biomasse wird dadurch erheblich beeinträchtigt. Darüberhinaus ist die Entwässerung der aus der Anlage entfernten Feststoffe bei der bekannten Anlage ungenügend, so dass zusätzlich Flüssigkeits/Feststoff-Trennvorrichtungen notwendig sind.

Aufgabe der Erfindung ist es, ein die Biomasse « schonendes » Verfahren zur Trennung von Biomasse und anorganischen Bestandteilen zu schaffen, bei dem mit Hilfe eines Hydrozyklons eine ausreichende Entwässerung der ausgeschiedenen Feststoffe erreicht wird, ohne dass relativ aufwendige Vorrichtungen zusätzlich erforderlich sind. Diese Aufgabe wird erfindungsgemäss dadurch gelöst, dass das zu trennende Schlamm/Wasser-Gemisch der Zulauföffnung durch den hydraulischen Reaktorvordruck zugeführt wird, und dass ferner der Oberlauf des Hydrozyklons in einer geschlossenen Leitung unter die Wasseroberfläche des Vorlagebehälters geführt wird.

Eine Vorrichtung zur Durchführung des Verfahrens mit einem Hydrozyklon zur Abtrennung anorganischer Bestandteile aus dem Schlamm des Reaktors, wobei der Zulauf des Hydrozyklons mit einem Schlammabzugsstutzen des Methan-Reaktors verbunden ist, wobei ferner der Oberlauf des Zyklons in einen mit Schlamm/Wasser-Gemisch mindestens teilweise gefüllten Vorlagebehälter mündet, ist dadurch gekennzeichnet, dass der Zulauf des Hydrozyklons unter der Niveauhöhe des Flüssigkeitsspiegels im Reaktor angeordnet ist, dass weiterhin eine vom Oberlauf des Hydrozyklons kommende geschlossene Verbindungsleitung in den Wasserinhalt des Vorlagebehälters eintaucht, und dass schliesslich eine Fördereinrichtung vorhanden ist, die saugseitig mit dem Wasserinhalt des Vorlagebehälters und druckseitig mit dem Methan-Reaktor verbunden ist.

Im Gegensatz zur bekannten Anlage, bei der der Oberlauf des Zyklons « offen » gegen die freie Atmosphäre ist, entsteht — durch die Förderung der Fördereinrichtung aus dem Vorlagebehälter — im Hydrozyklon ein Sog, durch den eine Flüssigkeitssäule aus diesem Hydrozyklon über den Oberlauf in den Vorlagebehälter hergestellt und aufrechterhalten wird; durch diesen Sog wird eine weitestgehende Trennung des Zulaufs in anorganische Feststoffe und Schlammwasser erreicht. So werden beispielsweise über 95 Vol.% des Zulaufs als Schlammwasser im Oberlauf abgeführt, während der Unterlauf nur einen Bruchteil von höchstens 5 Vol.% enthält.

Da die Dichte der Biomasse (etwa 1.05 g/cm) beträgt, also praktisch derjenigen des Wassers entspricht, gelangt die Biomasse dabei grösstenteils mit dem Wasser in den Oberlauf und in den Vorlagebehälter, aus dem sie zurück in den Methan-Reaktor gefördert wird. Analysen des Unterlaufs haben z. B. gezeigt, dass im als dickflüssige Paste austretenden Unterlauf der Wassergehalt etwa 15 bis 20 Vol.% beträgt, wobei eine weitere Analyse der etwa 85 % betragenden Feststoffanteile im Unterlauf ergeben haben, dass in diesem Feststoffanteil ein Restwasseranteil von 5 bis 7 % vorhanden ist, dass weiterhin etwa 90 % der mit dem Unterlauf ausgeschiedenen Feststoffe aus Kalzium-Carbonat bestehen, und dass schliesslich der Rest Sand und Biomasse ist.

Weiterhin ist der Energieaufwand für das neue Verfahren gering, da lediglich für den Antrieb einer Fördereinrichtung Energie zugeführt werden muss und zusätzliche Zentrifugen entfallen. Daher ist nur ein Bruchteil der Investionen erforderlich, die für die erwähnte bisherige Anlage notwendig sind.

Darüberhinaus findet eine mechanische Beanspruchung, vor allem eine Scherbeanspruchung, der Biomasse nur in der Fördereinrichtung zwischen dem Vorlagebehälter und dem Methan-Reaktor statt. Die Biomasse wird daher bei dem neuen Verfahren und in der neuen Vorrichtung sehr schonend behandelt und keinen hohen Belastungen ausgesetzt.

Um Verstopfungen des tangentialen Zulaufschlitzes am Hydrozyklon und/oder des Apex-Stopfens am Unterlaufaustritt aus dem Hydrozyklon zu vermeiden, ist es vorteilhaft, wenn das Schlamm/Wasser-Gemisch aus dem Methan-Reaktor durch eine rückspülbare Einrichtung zur Grobabscheidung von Feststoffpartikeln geführt wird, ehe es den Hydrozyklon erreicht ; entsprechend kann in der Vorrichtung dem Zulauf des Hydrozyklons eine Siebvorrichtung vorgeschaltet sein, deren Maschenweite kleiner ist als der Durchmesser der Apexstopfen-Oeffnung des Hydrozyklons.

Die unvermeidlichen Biomassen-Verluste werden bei dem neuen Verfahren umso geringer, je geringer der im Unterlauf abgeführte prozentuale Volumenanteil des Zulaufs ist. Dieser Volumenanteil lässt sich in gewissem Umfang variieren, wenn in den Hydrozyklon Apexstopfen mit unterschiedlichen Oeffnungsdurchmessern einsetzbar sind ; kleine Durchmesser des Apexstopfens ergeben dabei hohe Eindickungsgrade des Unterlaufs und damit geringe Verluste an Biomasse.

Erfahrungsgemäss erfolgt im Vorlagebehälter eine intensive Schaumbildung. Es ist daher zweckmässig, wenn die Eintauchtiefe des Oberlaufs in den Vorlagebehälter verändert werden kann, indem die Verbindungsleitung zwischen dem Oberlauf des Hydrozyklons und dem Vorlagebehälter flexibel und höhenverstellbar ausgebildet wird, um ein Eintauchen der Verbindungsleitung in die Flüssigkeit des Vorlagebehälters sicherzustellen.

Der Zulaufdruck zum Hydrozyklon — der im allgemeinen etwa 1 bar beträgt, womit in einem im Handel erhältlichen Hydrozyklon beispielsweise Beschleunigungen von einigen Hundert g erreicht werden — lässt sich in gewissem Masse variieren, wenn die Niveauhöhe des Zulaufs zum Hydrozyklon relativ zum Schlammabzugsstutzen des Methan-Reaktors veränderbar ist.

Im folgenden wird die Erfindung anhand eines Ausführrugsbeispiels im Zusammenhang mit der Zeichnung näher erläutert.

Fig. 1 zeigt schematisch eine Vorrichtung zur Durchführung des neuen Verfahrens ;

Fig. 2 gibt als Detail aus Fig. 1 eine Siebvorrichtung für das dem Zulauf des Hydrozyklons zugeführte Schlamm/Wasser-Gemisch wieder.

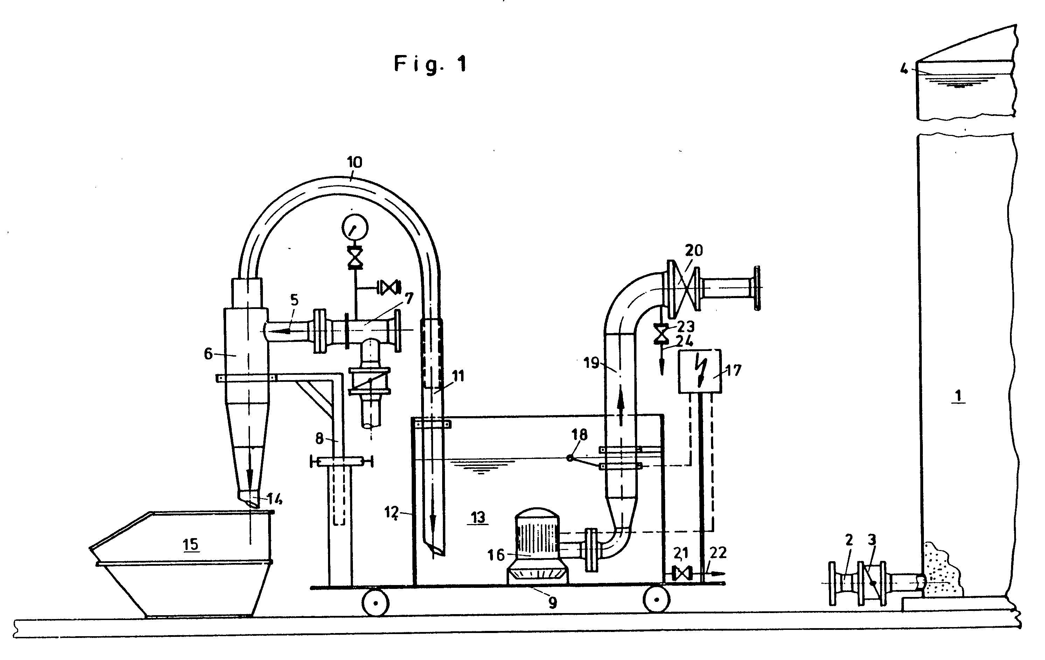

Der in Fig. 1 ganz rechts angedeutete Methan-Reaktor 1, dessen Flüssigkeitsniveau mit 4 bezeichnet ist, hat in Bodennähe einen Schlammabzugsstutzen 2, der mit einer Absperrklappe 3 verschliessbar ist. Der Abzugsstutzen 2 ist durch eine nicht dargestellte Zulaufleitung mit dem Zulaufeintritt 5 eines Hydrozyklons 6 verbunden ; diesem Zulaufeintritt 5 ist in Strömungsrichtung eine Siebvorrichtung 7 vorgelagert, die später näher beschrieben wird.

Der handelsübliche Hydrozyklon 6 ist an einem höhenverstellbaren Galgen B befestigt, der seinerseits auf einem Wagen 9 montiert ist, damit die Vorrichtung gegebenenfalls an einer Mehrzahl von Methan-Reaktor-Abzugsstutzen 2 angeschlossen werden kann.

Oben aus dem Hydrozyklon 6 führt eine flexible Verbindungsleitung 10, die in einem Schieberohr 11 endet, in den Wasserinhalt 13 eines Vorlagebehälters 12, der ebenfalls auf dem Wagen 9 montiert ist. Das Schieberohr 11 ist zur Einstellung der Eintauchtiefe der Verbindungsleitung 10 in dem Wasserinhalt 13 verschiebbar am Behälter 12 befestigt.

Die Austrittsöffnung für den Unterlauf des unten offenen Hydrozyklons 6 kann in bekannter Weise mit nicht dargestellten Apexstopfen versehen werden, deren Düsenbohrungen unterschiedliche Durchmesser von beispielsweise 3 bis 10 mm haben. Die Austrittsöffnung 14 ist über einem kippbaren Sammelbehälter 15 für das pastenartige, aus dem Schlamm/Wasser-Gemisch ausgeschiedene Material angeordnet.

Als Fördereinrichtung für die Rückführung von Schlamm/Wasser-Gemisch und damit von Biomasse aus dem Vorlagebehälter 12 in dem Methan-Reaktor 1 ist eine Tauchpumpe 16 im Behälter 12 vorgesehen, die durch einen symbolisch angedeuteten elektrischen Antrieb 17 angetrieben wird. Der Antrieb 17 der Tauchpumpe 16 kann von einem Schwimmer 18 ein- und ausgeschaltet werden, der vom Flüssigkeitsniveau im Vorlagebehälter 12 betätigt wird ; auch die Wirkungsweise der Schwimmersteuerung wird später noch beschrieben.

Die Druckleitung 19 der Pumpe 16 enthält ein einstellbares Drosselorgan 20, mit dem die Fördermenge der Tauchpumpe 16 variiert werden kann. Die Fortsetzung der Druckleitung 19 über das Drosselorgan 20 hinaus und ihr Anschluss an den Methan-Reaktor 1 sind nicht ausdrücklich dargestellt.

Der Vorlagebehälter 12 weist darüberhinaus eine, durch ein Ventil 21 absperrbare Entleerungsleitung 22 auf ; er kann - falls zur Bewahrung der anaeroben Eigenschaften der Biomasse notwendig oder sinnvoll — durch einen nicht dargestellten Deckel gegen die sauerstoffhaltige Atmosphäre abgeschirmt sein. Zur Ueberprüfung der Zusammensetzung und Qualität des in den Methan-Reaktor 1 zurückgeförderten Schlamm/Wasser-Gemisches zweigt aus der Druckleitung 19 eine mit einem Absperrorgan 23 versehene Probenentnahmeleitung 24 ab.

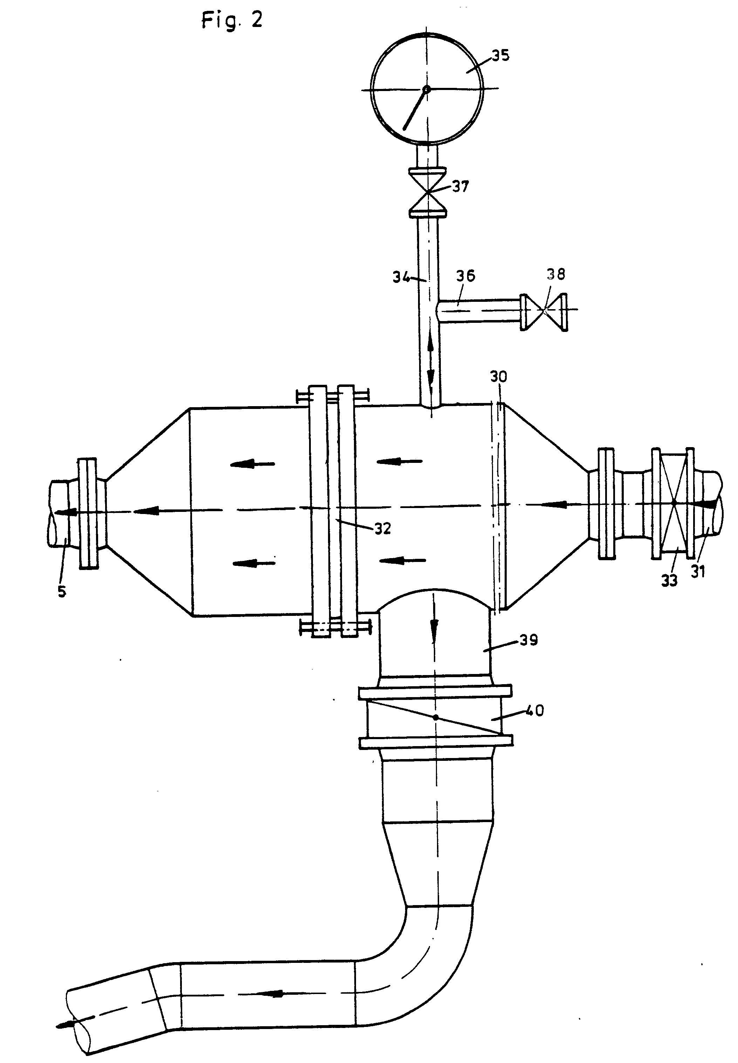

Die in Fig. 2 dargestellte Siebvorrichtung 7 enthält ein Siebgehäuse 30, das gegenüber der von rechts kommenden mit einer Zulaufklappe 33 versehenen Zulaufleitung 31 in seinem Volumen erweitert ist. In diesem Siebgehäuse 30 ist eine Siebplatte 32 angeordnet, die beispielsweise aus Edelstahl besteht, etwa 5 mm dick ist und als Sieböffnungen quadratische Löcher enthält, deren Diagonaldurchmesser kleiner ist als der Durchmesser der Bohrung im Apexstopfen des Hydrozyklons 6. Um einen Austausch von Siebplatten 32 bei der Verwendung von Apexstopfen mit unterschiedlichen Bohrungsdurchmessern zu vermeiden, ist es vorteilhaft, die Sieböffnungen nach der minimalen Düsenbohrung in den verwendeten Apexstopfen zu bemessen.

An das Siebgehäuse 30, das in der Ebene der Siebplatte 32 geteilt ist, schliesst sich nach links der Zulaufeintritt 5 des Hydrozyklons 6 an. Aus der Decke des Siebgehäuses 30 führt in Strömungsrichtung des Schlamm/Wasser-Gemisches während des Betriebs vor der Siebplatte 32 eine Messleitung 34, aus der eine Belüftungsleitung 36 in die freie Atmosphäre abzweigt, zu einem Manometer 35. Beide Leitungen 34 und 36 sind mit je einem Absperrhahn 37 und 38 versehen. Die Belüftungsleitung 36 hat die Aufgabe, in der Messleitung 34 ein Luftpolster zu erzeugen, das das Manometer vor Verunreinigungen schützt. Darüberhinaus dient sie dazu, beim Rückspülen ein schlagartiges Entleeren des Siebgehäuses 30 durch einströmende Luft zu erleichtern.

Aus dem Boden des Siebgehäuses 30 führt ein Spülablauf 39 über eine Absperrklappe 40, beispielsweise in den kippbaren Abfallsammelbehälter 15. Der Spülablauf 39 kann in seinem unteren Ende vorteilhafterweise schwenkbar ausgebildet sein.

Der Ablauf des erfindungsgemässen Verfahrens erfolgt mit der beschriebenen Vorrichtung auf folgende Weise : Zur Durchführung des Verfahrens ist der Abzugsstutzen 2 mit der Siebvorrichtung 7 verbunden, an der die Klappe 33 offen und die Organe 40 und 38 geschlossen sind. Gleichzeitig mit dem Oeffnen der Absperrklappe 3 wird auch die Tauchpumpe 16 in Betrieb gesetzt. Vorausgesetzt die Siebplatte 32 ist nicht durch grobe Verunreinigungen, insbesondere durch Kalzium-Carbonat-Plättchen verstopft, fliesst ein zu trennendes Schlamm/Wasser-Gemisch, angetrieben von dem hydraulischen Vordruck im Methan-Reaktor 1, aus diesem in den Hydrozyklon 6. Dort ist wegen der Absaugung von Schlamm/Wasser-Gemisch aus dem Vorlagebehälter 12 durch die Pumpe 16 ein Sog entstanden, durch die die spezifisch leichteren Teile des zu trennenden Gemisches, d. h. das Wasser und die Biomasse, zum weitaus grössten Teil als Oberlauf durch die Leitung 10 in den Vorlagebehälter 12 gelangen, während die schwereren Bestandteile in den Unterlauf des Hydrozyklons 6 fliessen. Wie erwähnt, wird der Unterlauf dabei soweit entwässert, dass über 95 % des Zulaufvolumens in den Oberlauf gehen ; da die Biomasse im Wasser weitgehend als Schwebeteilchen verteilt ist, gelangt der grösste Teil der Biomasse damit ebenfalls in den Vorlagebehälter 12 und wird aus diesem von der Pumpe 16 in den Methan-Reaktor 1 zurückbefördert.

Der weniger als 5 % der Zulaufvolumens betragende Rest wird zu einer pastenartigen Masse verdichtet und verlässt den Hydrozyklon 6 durch die Oeffnung 14. Er besteht zum grössten Teil (zu etwa 90 %) aus Kalzium-Carbonat ; der Rest ist Sand und Biomasse.

Steigt der durch das Manometer 5 ermittelte Vordruck vor der Siebplatte 32 unzulässig an, so muss die Siebvorrichtung gespült werden. Dazu schliesst man die Klappe 33 und den Hahn 37, während die Klappe 40 und der Hahn 38 geöffnet werden. Dadurch strömt das — wegen der Sogwirkung der Pumpe 16 — im Bogen der Verbindungsleitung 10 stehende Wasser durch den Zulaufeintritt 5 in Gegenrichtung zur normalen « Betriebsströmung » durch die Siebplatte 32 und spült die auf ihr sitzenden Verunreinigungen über den Spülablauf 39 in den Sammelbehälter 15. Diese Rückspülwirkung für das Siebgehäuse 30 wird dabei durch über die Leitung 36 einströmende Luft unterstützt und beschleunigt.

Gleichzeitig fliesst das unterhalb des Zulaufniveaus im Hydrozyklon 6 stehende Wasser durch die Unterlauföffnung 14 ebenfalls in den Behälter 15, so dass der Hydrozyklon 6 gleichfalls gespült wird.

Nach dem Schliessen der Klappe 40 und des Hahnes 39 und Oeffnung der Organe 33 und 37 kann der beschriebene « Normalbetrieb » der Vorrichtung fortgesetzt werden.

Die Steuerung des Flüssigkeitsniveaus im Vorlagebehälter 12 durch den Schwimmer 19 erfüllt dabei eine Sicherheitsfunktion ; durch sie wird nämlich verhindert, dass das Niveau im Vorlagebehälter 12 unzulässig weit absinkt und damit die Sogwirkung im Hydrozyklon 6 einen unteren Grenzwert unterschreitet. Der Schwimmer 18 schaltet dabei bei einem einstellbar festgelegten unteren Grenzniveau die Pumpe 16 ab. Innerhalb kurzer Frist steigt daraufhin die Flüssigkeit im Behälter 12, da der Zulauf aus dem Methan-Reaktor 1 zum Hydrozyklon 6 nicht unterbrochen wird, wieder an, worauf der Schwimmer 18 die Pumpe 16 wieder in Betrieb setzt.

Eine Einstellung der Pumpenleistung und damit der Sogwirkung für den Oberlauf des Hydrozyklons 6 wird dabei über das Drosselorgan 20 in der Druckleitung 19 vorgenommen.

**Patentansprüche**

1. Verfahren zum Trennen von Biomasse und anorganischen Bestandteilen aus dem Schlamm eines Methan-Reaktors (1) einer anaeroben Abwasseranlage, wobei das zu trennende Schlamm/Wasser-Gemisch in Bodennähe aus dem Reaktor (1) abgezogen und der Zulauföffnung (5) eines Hydrozyklons (6) zugeführt wird, wobei ferner der Oberlauf des Hydrozyklons (6) in einen mindestens Biomasse und Wasser enthaltenden Vorlagebehälter (12) geleitet wird, von dem aus schliesslich Schlamm/Wasser-Gemisch in den Reaktor (11) zurückgefördert wird, dadurch gekennzeichnet, dass das zu trennende Schlamm/Wasser-Gemisch der Zulauföffnung (5) durch den hydraulischen Reaktorvordruck zugeführt wird, und dass ferner der Oberlauf des Hydrozyklons (6) in einer geschlossenen Leitung (10) unter die Wasseroberfläche des Vorlagebehälters (12) geführt wird.

2. Verfahren nach Anspruch 1, dadurch gekennzeichnet, dass das Schlamm/Wasser-Gemisch aus dem Methan-Reaktor (1) durch eine rückspülbare Einrichtung (7) zur Grobabscheidung von Feststoffpartikeln geführt wird, ehe es den Hydrozyklon (6) erreicht.

3. Verfahren nach Anspruch 1 oder 2, dadurch gekennzeichnet, dass der durch den Unterlauf des Hydrozyklons (6) ausgeschiedenen Mengenanteil des Zulaufs verändert wird.

4. Verfahren nach einem der Ansprüche 1 bis 3, dadurch gekennzeichnet, dass die Eintauchtiefe des Oberlaufs in den Vorlagebehälter (12) verändert wird.

5. Vorrichtung zur Durchführung des Verfahrens nach Anspruch 1, mit einem Hydrozyklon (6) zur Abtrennung anorganischer Bestandteile aus dem Schlamm des Reaktors (1), wobei der Zulauf (5) des Hydrozyklons (6) mit einem Schlammabzugsstutzen (2) des Methan-Reaktors (1) verbunden ist, wobei ferner der Oberlauf des Zyklons (6) in einen mit Schlamm/Wasser-Gemisch mindestens teilweise gefüllten Vorlagebehälter (12) mündet, dadurch gekennzeichnet, dass der Zulauf (5) des Hydrozyklons (6) unter der Niveauhöhe (4) des Flüssigkeitsspiegels (4) im Reaktor (1) angeordnet ist, dass weiterhin eine vom Oberlauf des Hydrozyklons (6) kommende geschlossene Verbindungsleitung (10) in den Wasserinhalt (13) des Vorlagebehälters (12) eintaucht, und dass schliesslich eine Fördereinrichtung (16) vorhanden ist, die saugseitig mit dem Wasserinhalt (13) des Vorlagebehälters (12) und druckseitig mit dem Methan-Reaktor (1) verbunden ist.

6. Vorrichtung nach Anspruch 5, dadurch gekennzeichnet, dass dem Zulauf (5) des Hydrozyklons (6) eine Siebvorrichtung (7) vorgeschaltet ist, deren Maschenweite kleiner ist als der Durchmesser der Apexstopfen-Oeffnung des Hydrozyklons (6).

7. Vorrichtung nach einem der Ansprüche 5 oder 6, dadurch gekennzeichnet, dass die Niveauhöhe des Zulaufs (5) zum Hydrozyklon (6) relativ zum Schlammabzugsstutzen (2) des Methan-Reaktors (1) veränderbar ist.

8. Vorrichtung nach einem der Ansprüche 5 bis 7, dadurch gekennzeichnet, dass die Verbindungsleitung (10) zwischen dem Oberlauf des Hydrozyklons (6) und dem Vorlagebehälter (12) flexibel und höhenverstellbar ausgebildet ist.

## Claims

1. A method of separating biomass and inorganic constituents from the sludge from a methane reactor (1) in an anaerobic waste water plant, the sludge/water mixture for separation being withdrawn from the reactor (1) near the ground and fed to the inlet opening (5) of a hydrocyclone (6), the head flow from the hydrocyclone (6) being fed to a collecting tank (12) holding at least biomass and water, and a sludge/water mixture being returned from the collecting tank to the reactor (11), characterised in that the sludge/water mixture for separation is fed to the inlet opening (5) by the hydraulic flow pressure of the reactor, and the head flow from the hydrocyclone (6) is fed through a closed pipe (11) to beneath the surface of the water in the collecting tank (12).

2. A method according to claim 1, characterised in that the sludge/water mixture from the methane reactor (1) is fed through a back-washable device (7) for coarse separation of solid particles before reaching the hydrocyclone (6).

3. A method according to claim 1 or 2, characterised in that the proportion of the inflow separated by the tail flow of the hydrocyclone (6) is varied.

4. A method according to any of claims 1 to 3, characterised in that the depth of immersion of the head flow into the collecting tank (12) is varied.

5. Apparatus for performing the method according to claim 1, comprising a hydrocyclone (6) for separating inorganic constituents from the sludge from the reactor (1), the inlet (5) of the hydrocyclone (6) being connected to a sludge withdrawal spigot (2) of the methane reactor (1), and the head flow of the cyclone (6) terminating in a collecting tank (12) at least partly filled with sludge/water mixture, characterised in that the inlet (5) of the hydrocyclone (6) is disposed below the height (4) of the liquid level (4) in the reactor (1), a closed connecting pipe (10) coming from the head flow of the hydrocyclone (6) dips into the water (13) in the collecting tank (12), and a conveying device (16) is provided and connected on the suction side to the water (13) in the tank (12) and on the delivery side to the methane reactor (1).

6. Apparatus according to claim 5, characterised in that a filter device (7) is connected in front of the inlet (5) of the hydrocyclone (6) and has a mesh width smaller than the diameter of the apex plug opening of the hydrocyclone (6).

7. Apparatus according to claim 5 or 6, characterised in that the level of the inflow (5) to the hydrocyclone (6) is variable relatively to the sludge withdrawal spigot (2) of the methane reactor (1).

8. Apparatus according to any of claims 5 to 7, characterised in that the connecting pipe (10) between the head flow of the hydrocyclone (6) and the collecting tank (12) is flexible and vertically adjustable.

## Revendications

1. Procédé pour séparer la biomasse et les constituants inorganiques de la boue d'un réacteur à méthane (1) dans une installation de traitement anaérobie des eaux usées, dans lequel le mélange de boue et d'eau à séparer est soutiré près du fond du réacteur (1) et amené à l'orifice d'admission (5) d'un hydrocyclone (6), et en outre l'écoulement supérieur de l'hydrocyclone (6) est amené à un récipient collecteur (12) contenant au moins la biomasse et de l'eau, duquel finalement un mélange boue/eau est recyclé dans le réacteur (11), caractérisé en ce que le mélange boue/eau à séparer est amené à l'orifice d'admission (5) par la pression hydraulique d'alimentation du réacteur, et que l'écoulement supérieur de l'hydrocyclone (6) est en outre amené dans une conduite (10) fermée au-dessous de la surface de l'eau dans le récipient collecteur (12).

2. Procédé suivant la revendication 1, caractérisé en ce que le mélange boue/eau provenant du réacteur à méthane (1) passe par un dispositif (7) à rétro-lavage en vue de la séparation grossière de particules de matières solides avant de parvenir à l'hydrocyclone (6).

3. Procédé suivant la revendication 1 ou 2, caractérisé en ce qu'on fait varier la proportion quantitative de matière admise séparée par l'écoulement inférieur de l'hydrocyclone (6).

4. Procédé suivant l'une des revendications 1 à 3, caractérisé en ce qu'on fait varier la profondeur d'immersion de l'écoulement supérieur dans le collecteur (12).

5. Dispositif pour la mise en oeuvre du procédé suivant la revendication 1, comportant un hydrocyclone (6) pour la séparation de constituants inorganiques de la boue du réacteur (1), dans lequel l'admission (5) de l'hydrocyclone (6) est reliée avec un manchon d'extraction (2) de la boue du réacteur (1) à méthane, et en outre l'écoulement supérieur du cyclone (6) débouche dans un récipient collecteur (12) au moins partiellement rempli de mélange boue/eau, caractérisé en ce que l'admission (5) de l'hydrocyclone (6) se trouve au-dessous du niveau (4) de liquide dans le réacteur (1), qu'un conduit de liaison (10) fermé venant de l'écoulement supérieur de l'hydrocyclone (6) plonge en outre dans l'eau (13) contenue dans le récipient collecteur (12) et enfin qu'il est prévu un dispositif (16) de transport qui est en liaison du côté aspiration avec l'eau (13) contenue dans le récipient collecteur (12) et, du côté refoulement, avec le réacteur (1) à méthane.

6. Dispositif suivant la revendication 5, caractérisé en ce qu'un filtre (7), dont la largeur de maille est inférieure au diamètre de l'ouverture du bouchon d'extrémité de l'hydrocyclone (6), est placé en avant de l'admission (5) de l'hydrocyclone (6).

7. Dispositif suivant l'une des revendications 5 ou 6, caractérisé en ce qu'on peut faire varier le niveau de l'admission (5) à l'hydrocyclone (6) par rapport au tuyau d'évacuation de boue (2) du réacteur à méthane (1).

8. Dispositif suivant l'une des revendications 5 à 7, caractérisé en ce que la conduite de liaison (10) entre l'écoulement supérieur de l'hydrocyclone (6) et le récipient collecteur (12) est réalisée flexible et déplaçable en hauteur.

Fig. 1

0 163 749

Fig. 2